# DEMANDE DE BREVET EUROPEEN

(11) **EP 1 938 769 A1**
(43) Date de publication de la demande: **02.07.2008**
(21) Numéro de dépôt: 07291528.3
(22) Date de dépôt: 14.12.2007
(51) Int. Cl.: A61B 19/00, G06K 19/06, A61B 17/00, G06K 17/00

(54) **Instrument, tel qu'un instrument chirurgical**

(30) Priorité: 15.12.2006 FR 0655570
(71) Demandeur: Despres, Jean-Albert, 41300 Souesmes (FR); Normandin, Martine, 41300 Souesmes (FR)
(72) Inventeur: Despres, Jean-Albert, 41300 Souesmes (FR); Normandin, Martine, 41300 Souesmes (FR)
(74) Mandataire: Berger, Helmut

(57) **Abrégé**

L'invention concerne un instrument tel qu'un instrument chirurgical d'un type prédéterminé, défini par sa forme et sa fonction, parmi une pluralité d'instruments de types différents.

L'instrument est caractérisé en ce qu'il comporte une pièce (10) d'identification de l'instrument (4a), qui est rapportée sur la surface extérieure de l'instrument.

L'invention est utilisable pour le tri automatique d'instruments chirurgicaux.

## Description

L'invention concerne un instrument tel qu'un instrument chirurgical d'un type prédéterminé, défini par sa forme et sa fonction parmi une pluralité d'instruments de types différents.

Les instruments de ce genre, qui sont connus, peuvent être reconnus ou identifiés par les personnes devant les manipuler d'après leur aspect extérieur visuel, mais ne se prêtent pas à un tri pouvant être effectué de façon automatique.

L'invention a pour but de proposer des instruments dont le type peut être reconnu de façon automatique.

Pour atteindre ce but, un instrument selon l'invention est caractérisé en ce qu'il comporte une pièce d'identification de l'instrument, qui est rapportée sur la surface extérieure de l'instrument.

Selon une caractéristique de l'invention, l'instrument est caractérisé en ce que la pièce rapportée enferme un élément d'identification de l'instrument.

Selon une autre caractéristique de l'invention, l'instrument est caractérisé en ce que l'information d'identification réside dans la forme de l'élément d'identification.

Selon encore une autre caractéristique de l'invention, l'instrument est caractérisé en ce que l'élément d'identification est réalisé en un matériau relativement opaque aux rayons X, tandis que la pièce précitée et, le cas échéant, l'instrument sont en un matériau relativement transparent aux rayons X.

Selon encore une autre caractéristique de l'invention, l'instrument est caractérisé en ce que l'élément d'identification est de forme plate comportant des découpes de formes spécifiques constituant l'information d'identification.

Selon encore une autre caractéristique de l'invention, l'instrument est caractérisé en ce que l'élément d'identification est un corps de révolution.

L'invention sera mieux comprise, et d'autres buts, caractéristiques, détails et avantages de celle-ci apparaîtront plus clairement dans la description explicative qui va suivre faite en référence aux dessins schématiques annexés donnés uniquement à titre d'exemple illustrant plusieurs modes de réalisation de l'invention et dans lesquels :
- la figure 1 est une vue schématique, en perspective, d'une installation de reconnaissance d'instruments chirurgicaux selon l'invention, chacun pourvu d'une pièce d'identification, selon l'invention ;
- les figures 2 et 3 sont des vues schématiques d'un premier type d'instrument chirurgical selon l'invention, représentant deux versions de réalisation de la pièce d'identification rapporté ;
- les figures 4 A et 4 B sont des vues respectivement de dessus et latérales d'un deuxième type d'instrument chirurgical pourvu d'une pièce d'identification rapportée selon l'invention ;
- la figure 5 est une vue schématique d'un troisième type d'instrument chirurgical muni d'une pièce d'identification rapportée, selon l'invention ;
- les figures 6 à 8 montrent trois versions de pièces d'identification rapportées différentes, rapportées sur un quatrième type d'instrument chirurgical.

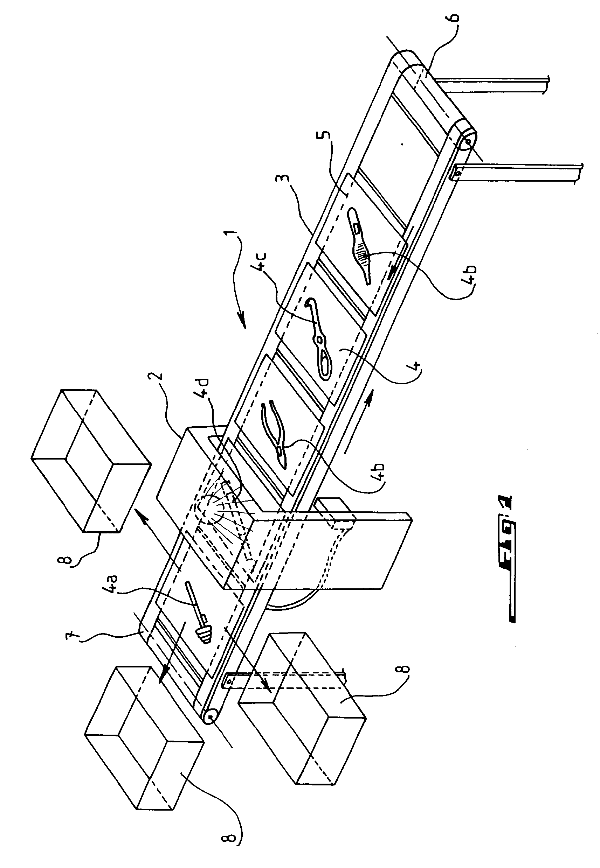

L'invention concerne des instruments chirurgicaux, de fonctions et de formes différentes, chacun pourvu d'un code d'identité de façon à être reconnaissables automatiquement, par exemple par une installation de reconnaissance telle que représentée sur la figure 1.

Cette installation, désignée par la référence générale 1 comporte essentiellement un poste 2 de reconnaissance des instruments chirurgicaux, pourvu d'un lecteur aux rayons X en forme de portique, et une bande transporteuse 3 destinée à faire passer les instruments chirurgicaux 4 de type différent et devant être identifiés par le poste de reconnaissance 2.

Dans l'exemple représenté, les instruments chirurgicaux 4 sont placés chacun sur un support 5 et amenés de l'extrémité d'admission 6 du convoyeur 3 à l'autre extrémité 7, ou, après le passage sous le portique de lecture 2, ils sont répartis dans différents récipients 8 selon des critères prédéterminés, par tous moyens appropriés connus.

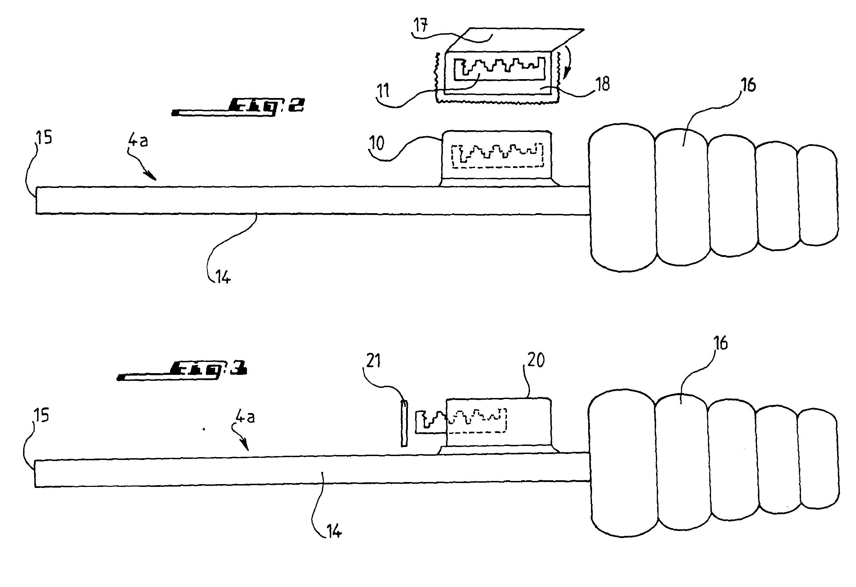

La figure 1 montre cinq types d'instrument chirurgical 4, qui sont donnés à titre d'exemple, et décrits ci-après plus en détail, en se référant aux figures 2 à 8.

Les quatre types d'instruments portent les références respectivement 4 a à 4 d.

La particularité des instruments chirurgicaux 4 selon l'invention réside dans le fait que chacun est pourvu d'une pièce rapportée 10 qui enferme, de façon étanche, un élément 11 porteur d'un code d'identification de l'instrument.

Les éléments 11 sont réalisés en un matériau relativement opaque aux rayons X, tandis que la partie d'enveloppe 12 et l'instrument sont fabriqués en un matériau relativement transparent aux rayons X ou au moins moins opaque à ces rayons que l'élément d'identification 11.

C'est grâce à cette différence de l'opacité que le lecteur aux rayons X 2 peut reconnaître la forme des éléments d'identification des instruments passant par ce lecteur, ces formes constituant l'information qui identifie l'instrument auquel il est rapporté.

Les figures 2 et 3 montrent un instrument d'un premier type, désigné par la référence 4 a, qui comporte une canule ou un tube d'aspiration 14 dont une extrémité, notée 15, est ouverte tandis que l'autre extrémité porte une poire d'aspiration 16 par exemple en caoutchouc.

La pièce d'identification 10 est rapportée sur la face extérieure du tube 14, en dessous de la poire 16, de toute façon appropriée, par exemple par soudage.

La pièce 10 comporte, comme on le voit sur la figure 2, une enveloppe extérieure 12 formée par deux volets 17, 18 susceptibles de pivoter autour d'une ligne de pliage 18 de façon à pouvoir enfermer, à l'état replié l'une sur l'autre, l'élément d'identification 11.

Cette dernière est formée par une plaquette dans laquelle sont pratiquées des découpes de formes particulières et qui constituent l'information permettant d'identifier l'instrument.

Etant donné que la pièce d'identification 10 est fixée sur la face extérieure de l'instrument, à un endroit approprié, elle n'est pas gênante pour la manipulation de l'instrument. L'élément d'identification 12 peut alors comporter non seulement l'information proprement dite d'identification de l'instrument, mais, le cas échéant, des informations supplémentaires utiles pour la manipulation et l'utilisation de l'instrument.

Par exemple, l'élément d'identification pourrait comporter des informations relatives au nombre de fois d'utilisation de l'instrument, aux précautions de manipulation et d'utilisation.

Par rapport à la figure 2, la figure 3 montre que l'enveloppe 12 pourrait aussi être formée par un boîtier parallélépipédique, relativement plate, notée 20, dans lequel l'élément d'identification 11 est inséré latéralement, après la fixation du boîtier sur le tube. Après l'insertion, le boîtier est fermé par un organe de fermeture 21.

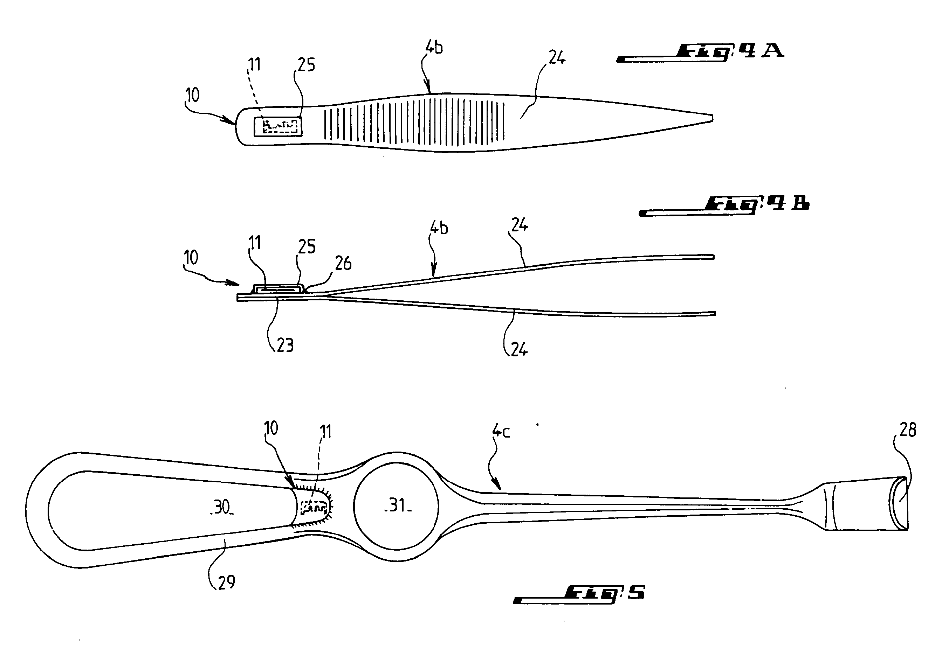

Les figures 4 A et 4 B montrent un deuxième type d'instrument chirurgical, désigné par la référence 4 b, à savoir une pince.

Cette pince est reconnaissable en tant que telle, grâce à la fixation de la pièce d'identification 10 sur la zone 23 de jonction des deux branches 24 de la pince.

La pièce présente la forme d'un boîtier sensiblement parallélépipédique plate 25, qui est fixée à plat sur la zone 23, par exemple par soudage, après insertion de l'élément d'identification 11. Pour assurer une fixation étanche, le cordon de soudage 26 s'étend tout autour du boîtier. Au lieu d'avoir un boîtier fermé, celui-ci pourrait aussi être en forme de cuvette, placée de façon qu'elle soit en appui sur la zone 23 par ses bords extérieurs libres.

La figure 5 illustre un troisième type d'instrument chirurgical désigné par la référence 4 c, à savoir un écarteur, pourvu d'une zone d'extrémité recourbée 28 et dont l'autre extrémité 29 est configurée comme partie d'actionnement pourvue d'un creux 30.

Dans ce cas, le boîtier d'identification 10 est placé dans l'extrémité du creux 20 près du centre 31 de l'instrument, c'est-à-dire dans une zone où cette pièce fixée, par exemple par soudage, n'est pas gênante pour l'utilisation de l'instrument.

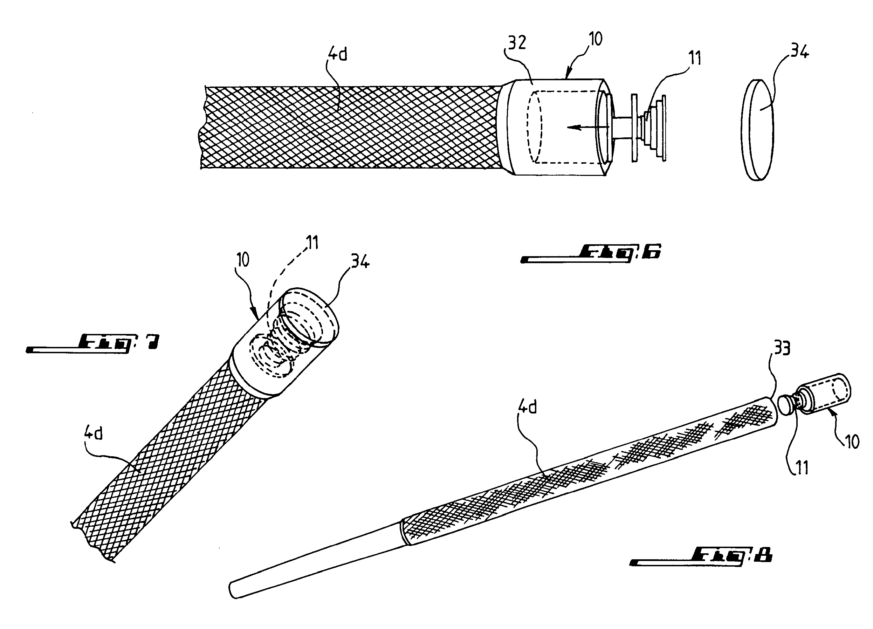

Les figures 6 à 8 montrent encore un autre type d'instrument chirurgical, désigné par la référence 4 d, à savoir une gouge à laquelle est rapportée une pièce d'identification 10, de forme cylindrique, fixée sur l'extrémité libre de manipulation 32.

Dans le cas des figures 6 et 7, le corps tubulaire 32 est rapporté à l'extrémité 33 de la gouge. Il est creux et destiné à recevoir l'élément d'identification 11 présentant alors la forme d'un corps de révolution de façon que l'information constituée par sa forme est lisible dans toutes positions angulaires de l'instrument. Après l'insertion de l'élément 10, le corps tubulaire 28 est fermé par un couvercle 34.

Dans le cas de la figure 8, l'élément d'identification constitue le prolongement axial du corps cylindrique 32 et est introduit dans l'extrémité alors creuse 33 de la gouge, la fixation du corps 32 se faisant de façon étanche sur cette extrémité.

Il ressort de la description qui précède, d'un certain nombre d'instruments chirurgicaux, donnée uniquement à titre d'exemple, que l'invention, en prévoyant la fixation d'une pièce rapportée, fixée à plat ou en forme de drapeau sur la face extérieure d'un instrument, permet une reconnaissance de l'instrument par un lecteur aux rayons X. Dans le cas de l'invention, la pièce d'identification est placée à un endroit approprié et peut avoir une forme adaptée à l'endroit de fixation.

Du fait de sa fixation à l'extérieur de l'instrument, il n'est pas nécessaire de donner à la pièce et ainsi à l'élément d'identification une taille la plus petite possible, si bien que l'élément d'identification pourrait porter des informations supplémentaires à celles strictement nécessaires pour l'identification de l'instrument, par exemple relatives à d'autres aspects liés notamment à l'utilisation de l'instrument.

## Revendications

1. Instrument tel qu'un instrument chirurgical d'un type prédéterminé, défini par sa forme et sa fonction, parmi une pluralité d'instruments de types différents, **caractérisé en ce qu'**il comporte une pièce (10) d'identification de l'instrument (4), qui est rapportée sur la surface extérieure de l'instrument, que la pièce rapportée (10) enferme un élément d'identification (11) de l'instrument qui comporte des découpes de formes spécifiques constituant l'information d'identification, et **en ce que** l'élément d'identification (11) est réalisé en un matériau relativement opaque aux rayons X, tandis que la pièce (10) précitée et, le cas échéant, l'instrument (4) sont en un matériau relativement transparent aux rayons X.

2. Instrument selon la revendication 1, **caractérisé en ce que** l'élément d'identification (11) est de forme plate.

3. Instrument selon la revendication 1, **caractérisé en ce que** l'élément d'identification (11) est un corps de révolution, les découpes étant des découpes circulaires.
